# EUROPEAN PATENT SPECIFICATION

(11) **EP 3 667 322 B1**
(45) Date of publication and mention of the grant of the patent: **01.07.2026**
(21) Application number: 19213734.7
(22) Date of filing: 05.12.2019
(51) Int. Cl.: G01N 33/569, C07K 14/445

(54) **SEROLOGICAL DETECTION OF PLASMODIUM ANTIBODIES**
SEROLOGISCHE DETEKTION VON PLASMODIUM-ANTIKÖRPERN
DÉTECTION SÉROLOGIQUE D'ANTICORPS DE PLASMODIUM

(30) Priority: 14.12.2018 EP 18212749
(43) Date of publication of application: 17.06.2020
(73) Proprietor: EUROIMMUN Medizinische Labordiagnostika AG, 23560 Lübeck (DE)
(72) Inventor: Probst, Christian, 23909 Ratzeburg (DE); Deeberg, Andrea, 23627 Groß Grönau (DE); Steinhagen, Katja, 23627 Groß Grönau (DE); Böthfür, Jana, 19217 Schlagsdorf (DE); Kruse, Tobias, 19217 Königsfeld/OT Klein Rünz (DE); Klemens, Oliver, 23562 Luebeck (DE); Menge, Babett, 21369 Nahrendorf (DE); Unger, Mandy, 20255 Hamburg (DE)

(56) References cited:
- WO-A1-2011/062637
- US-A1- 2009 226 445
- HAN H-J ET AL: "Epidermal growth factor-like motifs 1 and 2 of Plasmodium vivax merozoite surface protein 1 are critical domains in erythrocyte invasion", BIOCHEMICAL AND BIOPHYSICAL RESEARCH COMMUNICATIONS, ELSEVIER, AMSTERDAM, NL, vol. 320, no. 2, 23 July 2004 (2004-07-23), pages 563 - 570, XP004516668, ISSN: 0006-291X, DOI: 10.1016/J.BBRC.2004.06.008
- DIAS S ET AL: "Genetic diversity and recombination at the C-terminal fragment of the merozoite surface protein-1 of Plasmodium vivax (PvMSP-1) in Sri Lanka", INFECTION, GENETICS AND EVOLUTION, ELSEVIER, AMSTERDAM, NL, vol. 11, no. 1, 1 January 2011 (2011-01-01), pages 145 - 156, XP027561778, ISSN: 1567-1348, [retrieved on 20101215]

## Description

The present invention relates to diagnostically useful carrier comprising a means for specifically capturing an antibody to a *Plasmodium* p19 and a means for specifically capturing an antibody to a *Plasmodium* p33; a method comprising the step detecting in a liquid an antibody to a *Plasmodium* p19 and an antibody to a *Plasmodium* p33; a use of the diagnostically useful carrier according to any of claims 1 to 8 for identifying blood from a non-infected blood donor.

Malaria is a mosquito-borne disease caused by a parasite. Four species of malaria parasites can infect humans under natural conditions: *Plasmodium falciparum, P. vivax, P. ovale* and *P*. *malariae.* The first two species cause the most infections worldwide. *P. falciparum* is the agent of severe, potentially fatal malaria, causing an estimated 700,000 - 2.7 million deaths annually. *P. vivax* and *P. ovale* have dormant liver stage parasites referred to as hypnozoites which can reactivate and cause malaria several months or years after the infecting mosquito bite. *P. malariae* produces long-lasting infections and, if left untreated, can persist asymptomatically in the human host for years, even a lifetime.

More recently, *P. knowlesi,* whose natural hosts include crab-eating macaques (Macaca *fascicularis*) and pigtailed macaques, has been shown to infect humans and may be responsible for a significant number of human infections in Malaysia.

In humans, the parasites grow and multiply first in the liver cells and then in the red blood cells of the blood. In the blood, successive broods of parasites grow inside the red blood cells and destroy them, releasing daughter parasites that continue the cycle by infecting cells.

Malaria must be recognized promptly in order to treat the patient in time and to prevent further spread of infection in the community. Malaria should be considered a potential medical emergency and should be treated accordingly.

Although uncomplicated malaria is quite treatable and the symptoms are non-disabling, severe malaria occurs when *P. falciparum* infections are complicated by serious organ failures or abnormalities in the patient's blood or metabolism.

Usually malaria is transmitted by the bite of an infected *Anopheles* mosquito, but cases of transfusion-transmitted malaria (TTM) have been recorded. TTM can be acquired through blood components such as red cell concentrates, platelets, leucocytes, plasma and frozen red blood cells. TTM is rare in countries in which malaria is not endemic. Nevertheless, this disease has an impact on the resources of blood banks because over the last few years more and more prospective donors have been coming from malaria endemic areas either as tourists, immigrants or workers. Moreover, donated blood may be imported from countries where Malaria is endemic. Furthermore, the disease is currently re-emerging in many areas where it had been eradicated in the past and, consequently, the number of subjects coming from malaria areas has risen. It should also be borne in mind that climate change has recently altered the distribution of mosquitos and is likely to allow malaria to spread into new countries.

In many countries, whole blood donation is deferred for several years in potential donors who lived the first 5 years of their life in malaria endemic areas, and for several months in potential donors returning from an endemic area and those who have been infected previously.

This period may, however, be reduced if an immunological or molecular genomic test is negative prior to a blood donation, increasing the pool of blood donors. Therefore, there is significant demand for diagnostically useful immunoassays.

A major obstacle for establishing of reliable immunoassays has been the lack of soluble antigens. Even the proteolytically obtained fragments of merozoite surface protein (MSP) are large proteins and extremely difficult to obtain in large quantities of sufficient biological activity using recombinant methods as would be required for establishing a routine assay.

The state of the art describes a range of immunoassays for detecting a malaria infection in blood samples. Meyerhof, A. S. et al (2010) Clin*. and* Vacc. Immunol., page 1631-1638, and WO2009/111599 disclose ELISA, IFA and bead-based immunoassay for the detection of antibodies to 19 kDa carboxyterminal regions of the merozoite surface proteins 1 (MSP-1) from *P*. *falciparum, P. vivax, P. ovale* and *P*. *malariae.* Using an assay based on beads coated with all four antigens the authors reported an IgG detection sensitivity of 100%. However, the assay appears to have limited specificity, since samples from healthy blood donors gave positive results. p33 is disclosed, but not as an antigen for a diagnostic assay.

Suitable assays have been described in the state of the art and already yield reasonably good diagnostic reliabilities. However, owing to the large number of blood donors and samples to be test and the considerable shortage of donated blood, any further optimization towards 100% sensitivity and specificity should be considered a valuable contribution to the art.

WO2011/06237 discloses a vaccine to protect against malaria comprising a p19 region from *P. falciparum* MSP-1 in combination with selected segments of p33, which have immunogenic properties in a vaccine. The document does not disclose that such a mixture may be applied to a diagnostic immunoassay, let alone an increased specificity in the sense that false negative results may be avoided.

Lucchi *et al.* (2008) disclose different antibody responses to the merozoite surface protein-I complex in cerebral malaria patients in India. Patients suffering from cerebral malaria are in a coma, though, and therefore unlikely to give blood. In addition, there is no pointer to p33, let alone that it may be used to increase the sensitivity of a test based on p19 (Lucchi et al. (2008) Antibody responses to the merozoite surface protein-I complex in cerebral malaria patients in India, Malaria Journal, 7:121).

US2009/226445A1 is directed to anti-Plasmodium vaccine development and mentions recombinant polypeptides derived from Plasmodium p42, comprising p19 and selected fragments from p33 that enhance the immunogenicity of the p19 polypeptide.

Therefore, the problem underlying the present invention is to provide an immunoassay for detecting antibodies to *Plasmodium* proteins that yields more reliable results than state of the art assays, particularly in terms of specificity, sensitivity and overall diagnostic reliability. It is desirable that such an immunoassay may be performed in a high-throughput manner for the purpose of confirming that donated blood is not infected. The assay format should be based on soluble proteins obtainable using recombinant methods for high-throughput use and standardization. It should be possible to identify healthy donors and diagnose TTM in patients who have received a blood transfusion as well as to screen donated fractionated blood.

The problem underlying the present invention is solved by the subject matter of the independent and dependent claims.

In one aspect, the problem underlying the present invention is solved by a diagnostically useful carrier comprising all means from the group comprising
a means for specifically capturing an antibody to p19 from P. falciparum according to SEQ ID NO2,
a means for specifically capturing an antibody to p19 from P. vivax according to SEQ ID NO3,
a means for specifically capturing an antibody to p19 from P. ovale according to SEQ ID NO4 and
a means for specifically capturing an antibody to p19 from P. malariae according to SEQ ID NO5 and further comprising
a means for specifically capturing an antibody to p33 from P. *falciparum* according to SEQ ID NO8 and/or a means for specifically capturing an antibody to p33 from P. *vivax,* according to SEQ ID NO9, *most preferably* a means for specifically capturing an antibody to p33 from *P*. *falciparum*

In a preferred embodiment, the diagnostically useful carrier further comprises a means for specifically capturing an antibody to p19 from P. knowlesi.

In a preferred embodiment, at least two means, preferably all means are spatially separate from each other in the diagnostically useful carrier.

In a preferred embodiment, the carrier is selected from the group comprising a bead, preferably a magnetic and/or fluorescent bead, a test strip, a microtiter plate, a microarray, a solid polymer preferably derived from cellulose, a blot, preferably from the group comprising western blot, line blot and dot blot, a glass surface, a slide, preferably a glass slide for microscopy, a lateral flow device, a chromatography column, a biochip and a membrane, and is most preferably a microtiter plate.

In a preferred embodiment, the carrier is a mixture of beads on which the means are coated, wherein preferably each bead is coated with one type of means only.

In a preferred embodiment, the carrier is an ELISA microtiter plate with wells on which the means are coated, wherein preferably each well comprises one type of means only.

In a further aspect, the problem is solved by a method comprising the step of detecting in a liquid all antibodies from the group comprising
an antibody to p19 from P. falciparum according to SEQ ID NO2
an antibody to p19 from P. vivax according to SEQ ID NO3,
an antibody to p19 from P. ovale according to SEQ ID NO4 and
an antibody to p19 from P. malariae according to SEQ ID NO5 and one or more further antibodies, preferably all antibodies from the group comprising
an antibody to p33 from *P. falciparum* according to SEQ ID NO8 and
an antibody to p33 from *P. vivax* according to SEQ ID NO9.

In a preferred embodiment, at least an antibody to a *Plasmodium* p19 and an antibody to a *Plasmodium* p33 are detected in spatially separate reactions.

In a further aspect, the problem underlying the present invention is solved by a use of the diagnostically useful carrier according to the present invention for identifying a healthy blood donor.

Described herein is a use of a means for specifically capturing an antibody to a *Plasmodium* p19 and a means for specifically capturing an antibody to a *Plasmodium* p33 for the manufacture of an analytical device.

In a further aspect, the problem underlying the present invention is solved by a method for determining the ability, preferably capacity of a carrier according to the invention to bind to an antibody to p19 from P. falciparum according to SEQ ID NO2, an antibody to p19 from P. vivax according to SEQ ID NO3, an antibody to p19 from P. ovale according to SEQ ID NO4, an antibody to p19 from P. malariae according to SEQ ID NO5, an antibody to p33 from P. falciparum according to SEQ ID NO8 and an antibody to p33 from P. vivax according to SEQ ID NO9, comprising the step contacting the diagnostically useful carrier with a solution comprising a known concentration of at least one antibody to a *Plasmodium* p19 and a solution comprising a known concentration of at least one antibody to a *Plasmodium* p33.

In a further aspect, the problem underlying the present invention is solved by a kit comprising the diagnostically useful carrier according to the invention and a means for detecting an antibody to a sequence from the group comprising sequence from the group comprising SEQ ID NO2, SEQ ID NO3, SEQ ID NO4, SEQ ID NO5, SEQ ID NO6, SEQ ID NO7, SEQ ID NO8 and SEQ ID NO9 when bound to the diagnostically useful carrier, preferably a labeled secondary antibody,
wherein the kit preferably further comprises one or more, preferably all from the group comprising at least one from the group comprising sample dilution buffer, washing buffer, a positive control, preferably a recombinant antibody binding to one or more from the group comprising, SEQ ID NO2, SEQ ID NO3, SEQ ID NO4, SEQ ID NO5, SEQ ID NO6, SEQ ID NO7, SEQ ID NO8 and SEQ ID NO9, a negative control, one or more standard solutions comprising an antibody binding to one or more from SEQ ID NO1, SEQ ID NO2, to SEQ ID NO3, to SEQ ID NO4, to SEQ ID NO5, to SEQ ID NO6, to SEQ ID NO7, to SEQ ID NO8, to SEQ ID NO9 and to SEQ ID NO10 for preparing a calibration curve and a suitable water-tight vessel for contacting the diagnostically useful carrier with the sample in the presence of other liquids.

Described herein is a kit comprising a diagnostically useful carrier comprising a means for specifically capturing antibodies, preferably human antibodies to a Ig class, preferably IgG, and a polypeptide comprising a sequence from the group comprising SEQ ID NO1, SEQ ID NO2, SEQ ID NO3, SEQ ID NO4, SEQ ID NO5, SEQ ID NO6, SEQ ID NO7, SEQ ID NO8, SEQ ID NO9 and SEQ ID NO10 and a means for detecting said polypeptide when bound to the diagnostically useful carrier, preferably a label attached to the polypeptide or a labeled antibody binding to the polypeptide,
wherein the kit preferably further comprises one or more, preferably all from the group comprising at least one from the group comprising sample dilution buffer, washing buffer, a positive control, preferably a recombinant antibody binding to one or more from the group comprising SEQ ID NO1, SEQ ID NO2, SEQ ID NO3, SEQ ID NO4, SEQ ID NO5, SEQ ID NO6, SEQ ID NO7, SEQ ID NO8, SEQ ID NO9 and SEQ ID NO10, a negative control, one or more standard solutions comprising an antibody binding to one or more from SEQ ID NO1, SEQ ID NO2, to SEQ ID NO3, to SEQ ID NO4, to SEQ ID NO5, to SEQ ID NO6, to SEQ ID NO7, to SEQ ID NO8, to SEQ ID NO9 and to SEQ ID NO10 for preparing a calibration curve and a suitable water-tight vessel for contacting the diagnostically useful carrier with the sample in the presence of other liquids.

Described herein is a use of an antibody to a *Plasmodium* p33 or of a means for specifically capturing an antibody to a *Plasmodium* p33 for increasing the sensitivity of an immunoassay for the detection of *Plasmodium* in a liquid.

The present invention is based on the inventors' surprising finding that the combined detection of an antibody to a *Plasmodium* p19 and an antibody to a *Plasmodium* p33 greatly enhances the sensitivity and diagnostic reliability of an immunoassay for the detection of *Plasmodium.* Antigenic fragments of MSP-1 relating to p19 and, in particular, p33 may be obtained in a recombinant form in excellent qualities and quantities as would be required for setting up a high throughput assay.

The present invention relates to the detection of an antibody to *Plasmodium* polypeptides p19 and p33. In a preferred embodiment, the term *"Plasmodium* p19", as used herein, is a polypeptide comprising a sequence element comprising the sequence SEQ ID NO1, wherein a, b, c and d are each at least 1 and a is more preferably 2 to 10, most preferably 5, and b is more preferably 2 to 10, most preferably 5 to 6, and c is more preferably 5 to 15, most preferably 9 to 13, and d is preferably, or a variant thereof. Preferably said polypeptide comprises a fragment from the wild type polypeptide from which the p19 is naturally cleaved or a variant of said fragment, wherein the fragment has a length of no more than 500, preferably 400, 300, 250, 200, 150, 100 or 90 amino acids. For example, if the *Plasmodium* p19 is from *P. falciparum,* the fragment is from the MSP-1 from *P. falciparum.*

In a more preferred embodiment, the *Plasmodium* p19 is selected from the group comprising p19 from *P. falciparum* (SEQ ID NO2) and a variant thereof, *P. vivax* (SEQ ID NO3) and a variant thereof, *P. ovale* (SEQ ID NO4) and a variant thereof, *P. malariae* (SEQ ID NO5) and a variant thereof and *P. knowlesi* (SEQ ID NO6) and a variant thereof.

In a preferred embodiment, the term *"Plasmodium* p33", as used herein is a polypeptide comprising a sequence element comprising the sequence (SEQ ID NO7), wherein a and b are each at least 1, and a is more preferably 15 to 60, most preferably 21, 23, 29, 35 or 45, and b is more preferably 10 to 30, most preferably 16, 22 or 23, or a variant thereof. Preferably said polypeptide comprises a fragment from the wild type polypeptide from which the p19 is naturally cleaved or a variant of said fragment, wherein the fragment has a length of no more than 800, preferably 700, 600, 500, 450, 400, 350 or 320 amino acids. For example, if the *Plasmodium* p33 is from *P. falciparum,* the fragment is from the MSP-1 from *P. falciparum.*

In a more preferred embodiment, the *Plasmodium* p33 is selected from the group comprising p33 from *P. falciparum* (SEQ ID NO8) and a variant thereof, *P. vivax* (SEQ ID NO9) and a variant thereof, *P. ovale* (SEQ ID NO10) and a variant thereof, *P. malariae* (SEQ ID NO11) and a variant thereof and *P. knowlesi* (SEQ ID NO12) and a variant thereof. Whilst a combination of *Plasmodium* p33 and a *Plasmodium* p19 is preferred, it is possible to provide a carrier comprising the *Plasmodium* p33 and detect antibodies to it only.

The consensus sequences cited enable the person skilled in the art to use any p19 or p33 proteins from any further *Plasmodium strains,* which may be discovered in the future, or from similar organisms, which cause malaria or a similar disease or need to be detected to ensure the quality of donated blood for another reason.

The merozoite surface protein (MSP-1) is a large multiprotein complex exposed on the surface of the merozoites. During late schizogony, MSP-1 is proteolytically processed from its 190 kDa precursor into four major cleavage products: p83, p30, p38 and p42. During erythrocyte invasion, the p42 is further cleaved into p33 and p19, which is essential for invasion. The proteolytically processed MSP-1 appears to exist in association with the processed products of MSP-6 and MSP-7.

According to the present invention, a diagnostically useful carrier is provided which comprises a means for specifically capturing an antibody to a *Plasmodium* p19 and a means for specifically capturing an antibody to a *Plasmodium* p33. It comprises four means, each for specifically capturing an antibody to a *Plasmodium* p19 selected from the group comprising p19 from *P. falciparum* (SEQ ID NO2), *P. vivax* (SEQ ID NO3), *P. ovale* (SEQ ID NO4), *P. malariae* (SEQ ID NO5), and may comprise a fifth means for specifically capturing an antibody to a p19 from *P. knowlesi* (SEQ ID NO6). It comprises one or two means, each for specifically capturing an antibody to a *Plasmodium* p33 selected from the group comprising p33 from *P. falciparum* (SEQ ID NO8) and *P. vivax* (SEQ ID NO9). It may comprise two, three, four or five means, each for specifically capturing an antibody to a *Plasmodium* p33 selected from the group comprising p33 from *P. ovale* (SEQ ID NO10), *P. malariae* (SEQ ID NO11) and a variant thereof and *P. knowlesi* (SEQ ID NO12). In a more preferred embodiment, the term "means", as used herein, refers to any reagent or molecule capable of specifically capturing the respective antigen.

In a preferred embodiment, the term "specifically capturing an antibody", as used herein, refers to the ability to bind specifically to the antibody of interest to the effect that it is bound and removed from the liquid, whereas other antibodies, preferably of the same class or binding more strongly to a sequence other than the sequence to which the antibody to be captured binds, are essentially not bound and remain in the liquid. In a more preferred embodiment, native *Plasmodium* proteins and fragments thereof, for example MSP-6 and MSP-7 or processed products thereof are essentially absent. More preferably, the term "to capture specifically", also in variations such as "specifically capturing" or the like, as used herein, means that the binding reaction between the means and the antibody is stronger than a binding reaction characterized by a dissociation constant of 1 x 10⁻⁵ M, more preferably 1 x 10⁻⁷ M, more preferably 1 x 10⁻⁸ M, more preferably 1 x 10⁻⁹ M, more preferably 1 x 10⁻¹⁰ M, more preferably 1 x 10⁻¹¹ M, more preferably 1 x 10⁻¹² M, as determined by surface plasmon resonance using Biacore equipment at 25 °C in PBS buffer at pH 7.

In a more preferred embodiment, the means may comprise or may be a polypeptide or modified polypeptide or peptide or any other molecule mimicking a polypeptide or its specific amino acid sequence such that it is capable of specifically capturing the antibody of interest. In a preferred embodiment, the peptide or polypeptide comprises an epitope to the antibody to be captured or detected of at least 7, preferably 10, more preferably 15 amino acids. Said antigen, together with the insoluble carrier to which it is attached, may be separated from a reaction mixture, wherein it is contacted with a liquid, in a straightforward manner, for example by filtration, centrifugation or decanting. Said means may be immobilized in a reversible or irreversible manner. For example, the immobilization is reversible if the molecule interacts with the carrier via ionic interactions which may be masked by addition of a high concentration of salt or if the molecule is bound via a cleavable covalent bond. By contrast, the immobilization is irreversible if the molecule is tethered to the carrier via a covalent bond that cannot be cleaved in aqueous solution. The means may be indirectly immobilized, for example by immobilizing an antibody or other entity having affinity to the polypeptide, followed by addition of the polypeptide and formation of a polypeptide-antibody complex. The antibody captured may be detected using a labeled secondary antibody or a labeled means for specifically capturing the antibody to be detected.

In another preferred embodiment, the medical device is a microtiter plate comprising at least 8 wells that may be used for ELISA. At least one of the wells is directly or indirectly coated, for example using a coating antibody coupled to the plate which binds to the means, with at least one means for specifically capturing an antibody to a *Plasmodium* p19 and at least one means for specifically capturing an antibody to a *Plasmodium* p33. One well may be coated with a mixture comprising at least one means for specifically capturing an antibody to a *Plasmodium* p19 and at least one means for specifically capturing an antibody to a *Plasmodium* p33, preferably all such means used. At least 3, preferably 4, more preferably 5 calibrators are provided that comprise an antibody to the at least one *Plasmodium* p19 and/or the at least one *Plasmodium* p33 at a defined concentration and may be used to set up a calibration curve for semi-quantitative analysis. A secondary antibody comprising an enzymatically active label may be provided.

In another preferred embodiment, the diagnostically useful carrier comprises a means for specifically capturing any antibody of the class of antibody to be detected, preferably a means for specifically capturing all class, preferably IgG, IgA or IgM, preferably IgM class antibodies in the liquid, wherein the diagnostically useful carrier may be provided in combination with the antigen to which the antibody to be detected binds specifically such as a *Plasmodium* p19 or a polypeptide comprising a *Plasmodium* p33 or a variant thereof.

If that is the case, the assay is carried out such that the liquid is contacted with the carrier under conditions allowing binding to the carrier of all the antibodies in the liquid of the class of antibodies comprising the antibody to be detected, more specifically IgG class, preferably IgG antibody, followed by washing, followed by specific detection of the antibody to be detected. For this purpose, the carrier may be contacted with a labeled antigen to which the antibody binds such as a polypeptide comprising a *Plasmodium* p19 or a polypeptide comprising a *Plasmodium* p33 or a variant thereof.

The antigen or polypeptide comprising a *Plasmodium* p19 or p33 or antibody binding to a *Plasmodium* p19 or p33 or means for specifically capturing the antibody binding to a *Plasmodium* p19 or p33 may comprise a detectable label, preferably from the group comprising an enzymatically active, fluorescent, radioactive or luminescent, preferably chemiluminescent label or spin label. Alternatively the antigen may be dissociated from the antibody following specific binding and washing to detect its presence or absence.

Alternatively, a purified and labeled antibody may be used to detect a non-labeled antigen bound by the antibody to be detected which had in turn be captured by the means for specifically capturing all antibodies from the Ig class of the antibody to be detected, preferably IgG class, a method often referred to as bridge assay and described in various formats in the state of the art, for example US2018209973 or US5296347.

A competitive assay format may be used, wherein a complex comprising a *Plasmodium* p19 or a *Plasmodium* p33 or a variant thereof forms a complex with an antibody binding to it, following displacement of either the p19/p33 or the antibody from the complex, as detected using a label which is attached to the displaced or displacing p19/p33 or antibody.

If the diagnostically useful carrier is a bead, a mixture of beads, each carrying one type means for specifically capturing an antibody may be used, preferably a composition comprising a bead carrying a means for specifically capturing an antibody to a *Plasmodium* p19, preferably selected from the group comprising p19 from *P. falciparum* (SEQ ID NO2), *P. vivax* (SEQ ID NO3), *P. ovale* (SEQ ID NO4), *P. malariae* (SEQ ID NO5) and *P. knowlesi* (SEQ ID NO6), and a bead carrying a means for specifically capturing an antibody to a *Plasmodium* p33, preferably selected from the group comprising p33 from *P. falciparum* (SEQ ID NO8), *P. vivax* (SEQ ID NO9), *P. ovale* (SEQ ID NO10), *P. malariae* (SEQ ID NO11) and a variant thereof and *P. knowlesi* (SEQ ID NO12). Most preferably, the composition comprises ten different types of beads, each one carrying a different means from the group comprising means for specifically capturing an antibody to SEQ ID NO1, to SEQ ID NO2, to SEQ ID NO3, to SEQ ID NO4, to SEQ ID NO5, to SEQ ID NO6, to SEQ ID NO7, to SEQ ID NO8, to SEQ ID NO9 and to SEQ ID NO10. According to the present invention, a diagnostically useful carrier, preferably a bead, comprising a means for specifically detecting an antibody to a *Plasmodium* p33 is provided.

Various beads for numerous applications are commercially available, mainly based on carbohydrate, for example sepharose or agarose, or plastic. They contain active or activatable chemical groups such as carboxyl group, which can be utilized for the immobilization of reagents such as the means for specifically capturing an antibody. Preferably, the beads are beads having an average diameter of from 0.2 µm to 5 mm, from 0.5 µm to 1 mm, from 0.75 µm to 100 µm or from 1 µm to 10 µm. The beads can be coated with the means directly or via affinity ligands, for example biotin or glutathione. Preferably, the bead is provided in the form of an aqueous suspension having a bead content of from 10 to 90%, preferably from 20 to 80%, preferably from 30 to 70%, more preferably from 40 to 60% (w/w).

In a particularly preferred embodiment, the beads are paramagnetic beads, which can be easily concentrated on a surface with the aid of a magnet. For this purpose, commercial paramagnetic beads usually contain a paramagnetic mineral, for example iron oxide. A multiplicity of suitable paramagnetic beads is commercially available.

Alternatively, a bead comprising one or more means for specifically capturing an antibody to a *Plasmodium* p19 and a bead comprising one or more means for specifically capturing an antibody to a *Plasmodium* p33 may be used. A bead may comprise one or more means for specifically capturing an antibody to a *Plasmodium* p19 and one or more means for specifically capturing an antibody to a *Plasmodium* p33. More preferably, the bead may comprise ten different means for specifically capturing an antibody, one each for specifically capturing an antibody to SEQ ID NO1, SEQ ID NO2, SEQ ID NO3, SEQ ID NO4, SEQ ID NO5, SEQ ID NO6, SEQ ID NO7, SEQ ID NO8, SEQ ID NO9 and SEQ ID NO10, respectively.

The liquid used is any aqueous liquid that may comprise antibodies to be detected, preferably derived from blood. The liquid may preferably be a sample comprising antibodies from a subject. In a preferred embodiment, the sample is selected from the group comprising whole blood, serum, plasma, saliva, processed donated blood, preferably fractionated donated blood, more preferably fractions enriched in platelets or plasma or erythrocytes. Processed donated blood may comprise chemicals that stabilize it, for example by preventing clogging, preferably from the group comprising citrate, phosphate and dextrose.

However, the teachings of the present invention may not only be carried out using polypeptides, for example SEQ ID NO1, to SEQ ID NO2, to SEQ ID NO3, to SEQ ID NO4, to SEQ ID NO5, to SEQ ID NO6, to SEQ ID NO7, to SEQ ID NO8, to SEQ ID NO9 and to SEQ ID NO10, having the exact sequences referred to in this application explicitly, for example by function, name, sequence or accession number, or implicitly, but also using variants of such polypeptides.

In a preferred embodiment, the term "variant", as used herein, may refer to at least one fragment of the full length sequence referred to, more specifically one or more amino acid or nucleic acid sequence which is, relative to the full-length sequence, truncated at one or both termini by one or more amino acids. Such a fragment comprises or encodes for a peptide having at least 10, 15, 25, 50, 75, 100, 150, 200 or 250 successive amino acids of the original sequence or a variant thereof. The total length of the variant may be at 25, 30, 40, 50, 60, 70, 80, 90, 100, 150, 200 or 250 or more amino acids.

In another preferred embodiment, the term "variant" relates not only to at least one fragment, but also a polypeptide or a fragment thereof comprising amino acid sequences, preferably a fragment comprising at least 25, more preferably 50, more preferably 200 successive amino acids, that are at least 40, 50, 60, 70, 75, 80, 85, 90, 92, 94, 95, 96, 97, 98 or 99 % identical to the reference amino acid sequence referred to or the fragment thereof, wherein amino acids other than those essential for the biological activity, for example the ability to bind specifically to an antibody of interest, or the fold or structure of the polypeptide are deleted or substituted and/or one or more such essential amino acids are replaced in a conservative manner and/or amino acids are added or deleted such that the biological activity of the polypeptide is at least partially preserved. The state of the art comprises various methods that may be used to align two given nucleic acid or amino acid sequences and to calculate the degree of identity, see for example Arthur Lesk (2008), Introduction to bioinformatics, Oxford University Press, 2008, 3rd edition. In a preferred embodiment, the ClustalW software (Larkin, M. A., Blackshields, G., Brown, N. P., Chenna, R., McGettigan, P. A., McWilliam, H., Valentin, F., Wallace, I. M., Wilm, A., Lopez, R., Thompson, J. D., Gibson, T. J., Higgins, D. G. (2007): Clustal W and Clustal X version 2.0. Bioinformatics, 23, 2947-2948) is used applying default settings.

In a preferred embodiment, variants may, in addition, comprise chemical modifications, for example isotopic labels or covalent modifications such as glycosylation, phosphorylation, acetylation, decarboxylation, citrullination, hydroxylation and the like. The person skilled in the art is familiar with methods for the modification of polypeptides. Moreover, variants may also be generated by way of fusion with other known polypeptides or variants thereof, for example artificial linkers, preferably not derived from a *Plasmodium* polypeptide, affinity tags, other antigens and the like.

The variant of the polypeptide has biological activity. In a preferred embodiment such biological activity is the ability to bind to the respective antibody. In a preferred embodiment it comprises an epitope having the ability to bind to the respective antibody, wherein more preferably the epitope comprises a sequence comprising at least 5, 6, 7 or 8 amino acid residues. More specifically, a variant of SEQ X has the ability to bind specifically to an antibody binding to SEQ X from a sample from a patient infected with *Plasmodium,* wherein X is preferably selected from the group comprising SEQ ID NO1, SEQ ID NO2, SEQ ID NO3, SEQ ID NO4, SEQ ID NO5, SEQ ID NO6, SEQ ID NO7, SEQ ID NO8, SEQ ID NO9 and SEQ ID NO10.

The person skilled in the art is capable of designing variants having biological activity by starting from the sequence such as SEQ ID NO1, introducing modifications such as point mutations, truncations and the like and subsequently confirming that the variant still has biological activity by testing whether said variant binds to an antibody from such a sample. For the design of variants, the person skilled in the art can rely on a plethora of scientific publications detailing epitopes and antigenic properties of MSP1 proteins, for example Cheong, F. W., Gond, M. Y., and Lau, Y. L (2016) Identification and characterization of epitopes on Plasmodium knowlesi merozoite surface protein-142 (MSP-142) using synthetic peptide library and phage display library, Acta Trop., 154: 89-94; Lozano, J. M., Espejo, F., Ocampo, M., Salazar, L. M., Tovar, D., Barrera, N., Guzman, F., Patarroyo, M. E. (2004) Mapping the anatomy of a Plasmodium falciparum MSP-1 epitope using pseudopeptide-induced mono- and polyclonal antibodies and CD and NMR conformation analysis (2004) J. Struct. Biol. 148(1), 110-122; Parra, M., Hui, G., Johnson, A.H., Berzofsky, J. A., Roberts, T., Quakyi, I. A., Taylor, D. W. (2000) Characterization of conserved T- and B-cell epitopes in Plasmodium falciparum major merozoite surface protein 1 Infect Immun 68(5), 2685-91; Ghoshal, S., Gajendra, P., Datta Kanjilal, S., Mitra, M., Sengupta, S. (2018) Diversity analysis of MSP1 identifies conservative epitope organization in block 2 amidst high sequence variability in Indian Plasmodium falciparum isolates, Malar. J. 17(1)447; Sepulveda, M., Morais, C. G., Mourao, L. C., Freire, M. F., Fontes, C. J., Lacerda, M. V., Drakeley, C. J., Braga, E. M. (2016) Allele-specific antibodies to Palsmodium vivax merozoite surface protein-1: prevalence and inverse relationship to haemoglobin levels during infection, Malar J. 15(1), 559, Pusic, K.M., Hashimoto, C. N., Lehrer, A., Aniya, C., Clements, D.E., Hui, G. S. (2011) T cell epitope regions of the P. falciparum MSP1-33 critically influence immune responses and in vitro efficacy of MSP1-42 vaccines (2011) PLOS ONE, 6(9)e24782. Homologous epitopes from respective other Plasmodium strains can be identified by sequence alignment of known epitopes.

If a polypeptide is used as the means for specifically capturing an antibody, for example to SEQ ID NO1, said polypeptide, when used to carry out the teachings of the present invention, may be provided in any form and at any degree of purification, from tissues, fruits or cells comprising said polypeptide in an endogenous form, more preferably cells overexpressing the polypeptide, crude or enriched lysates of such cells, to purified and/or isolated polypeptide which may be essentially pure. In a preferred embodiment, the polypeptide is a native polypeptide, wherein the term "native polypeptide", as used herein, refers to a folded polypeptide, more preferably to a folded polypeptide purified from tissues or cells, more preferably from mammalian cells or tissues, optionally from non-recombinant tissues or cell. If a native polypeptide is used, it is preferably enriched compared to its natural state.

According to the present invention, the polypeptide may be a recombinant protein, wherein the term "recombinant", as used herein, refers to a polypeptide produced using genetic engineering approaches at any stage of the production process, for example by fusing a nucleic acid encoding the polypeptide to a strong promoter for overexpression in cells or tissues or by engineering the sequence of the polypeptide itself. The person skilled in the art is familiar with methods for engineering nucleic acids and polypeptides encoded (for example, described in Sambrook, J., Fritsch, E. F. and Maniatis, T. (1989), Molecular Cloning, CSH or in Brown T. A. (1986), Gene Cloning - an introduction, Chapman & Hall) and for producing and purifying native or recombinant polypeptides (for example Handbooks "Strategies for Protein Purification", "Antibody Purification", published by GE Healthcare Life Sciences, and in Burgess, R. R., Deutscher, M. P. (2009): Guide to Protein Purification). In another preferred embodiment, the polypeptide is an isolated polypeptide, wherein the term "isolated" means that the polypeptide has been enriched compared to its state upon production using a biotechnological or synthetic approach and is preferably pure, *i.e.* at least 60, 70, 80, 90, 95 or 99 percent of the polypeptide in the respective liquid consists of said polypeptide as judged by SDS polyacrylamide gel electrophoresis followed by Coomassie blue staining and visual inspection. Preferably any polypeptide on a carrier used as a means to capture an antibody is pure.

The antibody to a *Plasmodium* p19 or p33 may be detected in a liquid, preferably a sample from a subject. The is an organism producing antibodies, preferably IgM, IgA or IgG class antibodies, more preferably IgG class or equivalent allergy-related antibodies, more preferably a mammal, most preferably a human.

In a preferred embodiment, the one or more means for specifically capturing an antibody to a *Plasmodium* p19 and the one or more means for specifically capturing an antibody to a *Plasmodium* p33 are on separate carriers. This means that the means are not attached to a single carrier, but one or more carriers that are separate and/or separable without damaging them. For example, the means for specifically capturing an antibody to a *Plasmodium* p19 may be attached to a first test strip, and the means for specifically capturing an antibody to a *Plasmodium* p33 is attached to another test strip which is separate from the first test strip.

In another preferred embodiment, the one or more means for specifically capturing an antibody to a *Plasmodium* p19 and the means for specifically capturing an antibody to one or more *Plasmodium* p33 are on one, preferably covalently linked to one carrier, where it may be colocalized with another means or spatially separate from all other means. In a more preferred embodiment, the term "colocalized", as used herein, means that the two or more means are located on the carrier so closely that a signal associated with the detection of an antibody captured by one means cannot be distinguished from a signal associated with the detection of an antibody captured by another different means, preferably at least if the same kind of signal is generated. This means that the means are attached to one carrier which may not be disassembled, without damaging the carrier, such that the means are on separate carriers. For example, the means may be all coated on one test strip, particular in the form of a line blot. The carrier may be configured such that it is for incubating all these means with the same liquid at the same time. In other words, all binding reactions involving these means are in one pot.

The inventive teachings provide a kit, preferably for detecting *Plasmodium* infection or diagnosing Malaria, more preferably for diagnosing TTM. Such a kit is a container that comprises specific reagents required to practice the inventive method, in particular the diagnostically useful carrier according to the present invention, optionally in addition to one or more solutions or reagents required to practice the inventive method, preferably selected from or all from the group comprising sample dilution buffer, washing buffer and buffer comprising a means for detecting any specifically captured antibody, such as a secondary antibody and optionally a means for detecting the latter. Furthermore, it may comprise instructions detailing how to use the kit. It may comprise the diagnostically useful carrier for contacting the inventive polypeptide with a bodily fluid sample from a subject, preferably a human subject, for example a line blot. Furthermore, the kit may comprise a positive control, for example a recombinant antibody known to bind to one or more from the group comprising SEQ ID NO1, SEQ ID NO2, SEQ ID NO3, SEQ ID NO4, SEQ ID NO5, SEQ ID NO6, SEQ ID NO7, SEQ ID NO8, SEQ ID NO9 and SEQ ID NO10, and a negative control, for example a protein having no detectable affinity to a *Plasmodium* p19 or p33 such as bovine serum albumin. Finally, such a kit may comprise one or more standard solutions comprising an antibody binding to one or more from SEQ ID NO1, SEQ ID NO2, SEQ ID NO3, SEQ ID NO4, SEQ ID NO5, SEQ ID NO6, SEQ ID NO7, SEQ ID NO8, SEQ ID NO9 and SEQ ID NO10 for preparing a calibration curve, wherein the absolute or relative concentration of the antibody in each standard solution may preferably be known. The kit may comprise a means or reagent for detecting a captured antibody. The kit may comprise a purified polypeptide comprising one sequence from the group comprising SEQ ID NO1, SEQ ID NO2, SEQ ID NO3, SEQ ID NO4, SEQ ID NO5, SEQ ID NO6, SEQ ID NO7, SEQ ID NO8, SEQ ID NO9 and SEQ ID NO10, which polypeptide may optionally be labeled. The kit may also comprise a washing solution. The kit may comprise a suitable water-tight vessel for contacting the diagnostically useful carrier with the sample in the presence of other liquids such as a reaction buffer. For example, a line blot may be provided in or in combination in an incubation tray. Suitable vessels are described in the state of the art, for example EP3025780 or EP3025779).

According to the present invention, a means or reagent for detecting a captured antibody may be provided or used to detect a captured antibody to a *Plasmodium* p19 and to detect an antibody to a *Plasmodium* p33. This means or reagent may be a secondary antibody binding to the constant region of an antibody of the class of interest. For example, if a captured human IgG class antibody is to be detected, an antibody binding specifically to the constant region of human IgG class antibodies may be used. The means or reagent may be directly or indirectly labeled. It binds specifically, which preferably means that the binding reaction between the means and the captured antibody is stronger than a binding reaction characterized by a dissociation constant of 1 x 10⁻⁵ M, more preferably 1 x 10⁻⁷ M, more preferably 1 x 10⁻⁸ M, more preferably 1 x 10⁻⁹ M, more preferably 1 x 10⁻¹⁰ M, more preferably 1 x 10⁻¹¹ M, more preferably 1 x 10⁻¹² M, as determined by surface plasmon resonance using Biacore equipment at 25 °C in PBS buffer at pH 7.

The products and methods according to the present invention may be used for detecting a *Plasmodium* infection or contamination in a liquid such as donated blood *in vitro.* The donated blood is preferably from a donor whose identity is unknown. The blood is likely to be contaminated with *Plasmodium* if an antibody to at least one *Plasmodium* p19 or at least one *Plasmodium* p33 is detected.

They may also be used for diagnosing a disease which is a *Plasmodium* infection, preferably Malaria, more preferably TTM in a subject. In a preferred embodiment, the term "diagnosis", as used herein, refers to any kind of procedure aiming to obtain information instrumental in the assessment whether a patient suffers or is likely or more likely than the average or a comparative subject, the latter preferably having similar symptoms, to suffer from certain a disease or disorder in the past, at the time of the diagnosis or in the future, to find out how the disease is progressing or is likely to progress in the future or to evaluate the responsiveness of a patient with regard to a treatment. In other words, the term "diagnosis" comprises not only diagnosing, but also prognosticating and/or monitoring the course of a disease or disorder. The subject is likely to suffer from the disease if an antibody to at least one *Plasmodium* p19 or at least one *Plasmodium* p33 is detected in his liquid.

Therefore, the term "diagnosis" does preferably not imply that the diagnostic methods or agents according to the present invention will be definitive and sufficient to finalize the diagnosis on the basis of a single test, let alone parameter, but may refer to a contribution to what is referred to as a "differential diagnosis", *i.e.* a systematic diagnostic procedure considering the likelihood of a range of possible conditions on the basis of a range of diagnostic parameters. In a preferred embodiment, the term "diagnosis" means that the method or product or use may be used for aiding in the diagnosis of a disease or identifying a subject a risk of suffering from a disease. The term "diagnosis" may also refer to a method or agent used to choose the most promising treatment regime for a patient. In other words, the method or agent may relate to selecting a treatment regimen for a subject.

The present invention relates to a method comprising the step detecting in a liquid from a subject the presence or absence of an antibody a *Plasmodium* p19 and a *Plasmodium* p33. Such a method may comprise the steps a) providing a liquid, preferably a sample from donated processed and/or fractionated blood or sample from a subject, b) contacting the liquid with the diagnostically useful carrier according to the present invention under conditions compatible with the formation of a complex comprising the diagnostically useful carrier and the antibody, more specifically the means for specifically capturing the antibody and the antibody, c) isolating any said complex, for example by removing the liquid, d) optionally washing said complex, and e) detecting said complex, optionally after contacting with a *Plasmodium* p19 and/or *Plasmodium* p33, which may be labeled. The method is preferably an *in vitro* method.

The detection of the antibody or complex for the prognosis, diagnosis, methods or kit according to the present invention comprises the use of a method selected from the group comprising immunodiffusion techniques, immunoelectrophoretic techniques, light scattering immunoassays, agglutination techniques, labeled immunoassays such as those from the group comprising radiolabeled immunoassays, enzyme immunoassays such as colorimetric assays, chemiluminscence immunoassays and immunofluorescence techniques. In a preferred embodiment, the complex is detected using a method selected from the group comprising immunodiffusion techniques, immunoelectrophoretic techniques, light scattering immunoassays, agglutination techniques, labeled immunoassays from the group comprising radiolabeled immunoassays, chemiluminscence immunoassays and immunofluorescence techniques. The person skilled in the art is familiar with these methods, which are also described in the state of the art, for example in Zane, H. D. (2001): Immunology - Theoretical & Practical Concepts in Laboratory Medicine, W. B. Saunders Company, in particular in Chapter 14.

In another preferred embodiment, the prognosis, diagnosis, methods or kit in line with the inventive teachings contemplate the use of indirect immunofluorescence. The person skilled in the art is familiar with such techniques, which are described in the state of the art (US4647543; Voigt, J., Krause, C., Rohwäder, E, Saschenbrecker, S., Hahn, M., Danckwardt, M., Feirer, C., Ens, K, Fechner, K, Barth, E, Martinetz, T., and Stöcker, W. (2012), Automated Indirect Immunofluorescence Evaluation of Antinuclear Autoantibodies on HEp-2 Cells," Clinical and Developmental Immunology, vol. 2012, doi:10.1155/2012/65105; Bonilla, E., Francis, L., Allam, F., et al., Immuno-fluorescence microscopy is superior to fluorescent beads for detection of antinuclear antibody reactivity in systemic lupus erythematosus patients, Clinical Immunology, vol. 124, no. 1, pp. 18-21, 2007). Suitable reagents, devices and software packages are commercially available, for example from EUROIMMUN, Lübeck, Germany.

In many cases detecting, preferably meaning detecting the absence or presence of an antibody, optionally meaning determining whether the concentration of the antibody is beyond a certain threshold preferably as set by measurement using ELISA, in the liquid, is sufficient for the diagnosis. If the antibody can be detected, this will be information instrumental for the clinician's diagnosis and indicates an increased likelihood that the patient suffers from a disease. In a preferred embodiment, the relative concentration of the antibody in the serum, compared to the level that may be found in the average healthy subject, may be determined. In a preferred embodiment, the term "detecting the presence", as used herein, means that it is sufficient to check whether a signal sufficiently beyond any background level may be detected using a suitable complex detection method that indicates that the antibody of interest is present or more antibody of interest is present than would be in a healthy subject.

In a preferred embodiment, the absence or presence of one or more antibodies to a *Plasmodium* p19 and one or more antibodies to a *Plasmodium* p33 is detected simultaneously, *i.e.* at the same time.

In a preferred embodiment, the absence or presence of one or more antibodies to a *Plasmodium* p19 and one or more antibodies to a *Plasmodium* p33 is detected in spatially separate reactions, more preferably in different reaction mixtures in separate vessels.

If more than one antibody is to be detected, the method may be carried out in a one-pot reaction. Preferably, the term "one-pot reaction", as used herein, means that two or more, preferably all reactions carried out for the purpose of detecting the presence or absence of an antibody are carried out in the same reaction mixture in one reaction vessel, without physical barriers between the reactions, by contrast to experimental settings contemplating that at least two reactions are carried out in separate solutions and reaction vessels.

According to the present invention, an antibody to a *Plasmodium* p19 and an antibody to a *Plasmodium* p33 and optionally additional antibodies are detected. In a preferred embodiment, this means that it is detected whether at least one of these antibodies is present, without distinguishing to which of the antigens the antibody binds or whether only one or more than one antibodies are present. This may be done using means for specifically capturing which are co-localized, i.e. not spatially separated. In another preferred embodiment, this means that it is detected for each antigen such as a *Plasmodium* p19 and a *Plasmodium* p33 whether an antibody binding to the antigen is present. In any event, it is important to ensure that antibodies who bind to an antigen other than a *Plasmodium* p19 and *Plasmodium* p33 are not mistakenly taken as antibody binding to a *Plasmodium* p19 and *Plasmodium* p33 to avoid false positive results.

In a preferred embodiment, the invention is used to check on an analytical device. In a preferred embodiment, the term "analytical device" refers to a device made for non-diagnostic purposes, in particular for detecting at least one antibody to a *Plasmodium* p19 and at least one antibody to a *Plasmodium* p33 for screening donated blood.

The invention provides a use of one or more means for specifically capturing an antibody to a *Plasmodium* p19 and one or more means for specifically capturing an antibody to a *Plasmodium* p33 for the manufacture of a kit for the diagnosis of a disease or for screening donated blood for infection or contamination with *Plasmodium,* wherein preferably a diagnostic or analytical assay with an increased sensitivity is provided. Such manufacture may relate to a method comprising the step immobilizing on a diagnostically useful carrier one or more means for specifically capturing an antibody to a *Plasmodium* p19 and one or more means for specifically capturing an antibody to a *Plasmodium* p33.

### Sequences:

Reference is made to a range of novel polypeptides, more specifically
**SEQ ID NO1 (*Plasmodium* p19 consensus motif)** 
HXC(Xₐ)P(E/D)NXXCXRXXDXXEXXXCLLXXKXXXXXCV(X_{b})CXXNNGGCXXXAXC(X_{c})CXC(
X_{d})F(D/E)GXFCX,
 wherein each of a, b, c and d is at least 1.
**SEQ ID NO2 (*P. falciparum* p19)** 
NISQHQCVKKQCPENSGCFRHLDEREECKCLLNYKQEGDKCVENPNPTCNENNGGCDAD
AKCTEEDSGSNGKKITCECTKPDSYPLFDGIFCS
**SEQ ID NO3 (*P. vivax* p19)** 
TMSSEHTCIDTNVPDNAACYRYLDGTEEWRCLLTFKEEGGKCVPASNVTCKDNNGGCAPE
AECKMTDSNKIVCKCTKEGSEPLFEGVFCS
**SEQ ID NO4 (*P. ovale* p19)** 
SMGSKHKCIDITYPDNAGCYRFSDGREEWRCLLNFKKVGETCVPNNNPTCAENNGGCDPT
ADCAESENNKITCTCTGQNESFFEGVFCG
**SEQ ID NO5 (*P. malariae* p19)** 
NISAKHACTETKYPENAGCYRYEDGKEVWRCLLNYKLVDGGCVEDEEPSCQVNNGGCAPE
ANCTKGDDNKIVCACNAPYSEPIFEGVFCG
**SEQ ID NO6 (*P. knowlesi* p19)** 
NMSSAHKCIDTNVPENAACYRYLDGTEEWRCLLGFKEVGGKCVPASITCEENNGGCAPEA
ECTMDDKKEVECKCTKEGFEPLFEGVFCS
SEQ ID NO7 (*P*. p33 consensus motif) 
YXVXYXKPLAGXYXXXXKQXXXXXXXFXXNXXXXLXXRXKKRXYFLXVLXSXLXXXKXXXSX
XYXIXDXXKLLXXXXKXXLXXSYXYIXXXXXXDXXXAXXGXXYYXKXXXXYKXXL(Xa)KXXPFL
XXXXXXYXXLXXXXXXXXXXLKXXXXXCXXEKXXXXXXXXKLXXXXXXDXXXXXXK(Xb)LXX
XXXXXXXXXXXXXSXL,
 wherein each of a and b is at least 1
SEQ ID NO8 (*P. falciparum p33)* 
AISVTMDNILSGFENEYDVIYLKPLAGVYRSLKKQIEKNIFTFNLNLNDILNSRLKKRKYFLDVL
ESDLMQFKHISSNEYIIEDSFKLLNSEQKNTLLKSYKYIKESVENDIKFAQEGISYYEKVLAKYK
DDLESIKKVIKEEKEKFPSSPPTTPPSPAKTDEQKKESKFLPFLTNIETLYNNLVNKIDDYLINL
KAKINDCNVEKDEAHVKITKLSDLKAIDDKIDLFKNPYDFEAIKKLINDDTKKDMLGKLLSTGLV
QNFPNTIISKLIEGKFQDML
SEQ ID NO9 (*P. vivax* p33) 
DQVTTGEAESEAPEILVPAGISDYDVVYLKPLAGMYKTIKKQLENHVNAFNTNITDMLDSRLK
KRNYFLEVLNSDLNPFKYSSSGEYIIKDPYKLLDLEKKKLIGSYKYIGASIDMDLATANDGVT
YYNKMGELYKTHLDGVKTEIKKVEDDIKKQDEELKKLGNVNSQDSKKNEFIAKKAELEKYLP
FLNSLQKEYESLVSKVNTYTDNLKKVINNCQLEKKEAEITVKKLQDYNKMDEKLEEYKKSEK
KNEVKSSGLLEKLMKSKLIKENESKEILSQLLNVQTQLL
SEQ ID NO10 (*P. ovale* p33) 
AQDKDDNILNEITNEYEVVYVKPLAGVYKTLKKQLEAHVTAFHSNVTNMLESRLKKRNYFLE
VLNSDLTQYKHATSDNYVIRDAYKLLDFEKKKKLLSSYKYIKDSVEKDVEIATDGIDYYEKMA
ALYKTYLESVNAQVDAIDKTGDDATKATNKKFLPFLASINAMYETLLEKVNTYNSQLKSSLNS
CQLEKIRVGIVVDKLNDYVMFDEKLEELKSSKEKDLTKLYKDIDTSNIINKLKRSGFVDTDESK
KLLSELLDVDSAQLL
SEQ ID NO11 (*P. malariae* p33) 
AETKQDDNILKPITNEYEVLYIKPLAGVYRVLRKQIGDQIDAFNSNLTNALDTRKKKRTYFLDV
LNSDLIQFKHATSDSYIIKDPYKLLDVDKKAKLIGSYKYIVSAIEKDITSAENGVEYYDKMTKLY
KTQLEAVKSAIAEAQKEGDKKTENEKYIPFLTNMQTLYENLLNKINGNIINLKTLITNCNLEKDA
VNITISKLTEYSKFDEKIEMFKNSKNEKDIASSGILDILKQKGLVNKNESTKIISELLGVDSNALL
SEQ ID NO12 (*P. knowlesi* p33) 
AENAQPENIVPEGINEYEVVYIKPLAGMYKSIKKQLENHVAAFNTNITDMLDSRLKKRNYFLD
VLDSELNPFKYSSSGEYIIKDPYKLLDLEQKKKLLGSYQYIGASVDKDLITAKDGMEYYNKMG
ELYKQHLEAVNAQIKEIEASVPGEQSQLNAQKEELKKYLPFLNSIQKEYESLVNMAHTYKENL
KKFINNCQIEKKETEIIVKKLEDYTKIDENLEIYKKSKKESDVRSSGLLEKLKNSKLINEEESKK
VLSQLLNVQTQML
**SEQ ID NO 13 (His-tagged p19 from *P. falciparum*)**
   **>P6527_His-MSP-1-p19_P.falciparum_B140110RD** 
MSHHHHHHHHSMNLAKTVLSNLLDGNLQGMLNISQHQCVKKQCPENSGCFRHLDEREEC
KCLLNYKQEGDKCVENPNPTCNENNGGCDADAKCTEEDSGSNGKKITCECTKPDSYPLFD
GIFCS
**SEQ ID NO 14 (His-tagged p33 from *P. falciparum*)**
   **>P16464_His-MSP-1-p33_P.falciparum_B180806RB** 
MSHHHHHHHHSMAISVTMDNILSGFENEYDVIYLKPLAGVYRSLKKQIEKNIFTFNLNLNDILN
SRLKKRKYFLDVLESDLMQFKHISSNEYIIEDSFKLLNSEQKNTLLKSYKYIKESVENDIKFAQ
EGISYYEKVLAKYKDDLESIKKVIKEEKEKFPSSPPTTPPSPAKTDEQKKESKFLPFLTNIETL
YNNLVNKIDDYLINLKAKINDCNVEKDEAHVKITKLSDLKAIDDKIDLFKNPYDFEAIKKLIN
**SEQ ID NO 15 (His-tagged p19 from *P. vivax*)**
   **>P6526_His-MSP-1-p19_P.vivax_B140110RE** 
MSHHHHHHHHSMENESKEILSQLLNVQTQLLTMSSEHTCIDTNVPDNAACYRYLDGTEEW
RCLLTFKEEGGKCVPASNVTCKDNNGGCAPEAECKMTDSNKIVCKCTKEGSEPLFEGVFCS
**SEQ ID NO 16 (His-tagged p33 from *P. vivax*)**
   **>P16463_His-MSP-1-p33_P.vivax_B180806RA** 
MSHHHHHHHHSMSDQVTTGEAESEAPEILVPAGISDYDVVYLKPLAGMYKTIKKQLENHVN
AFNTNITDMLDSRLKKRNYFLEVLNSDLNPFKYSSSGEYIIKDPYKLLDLEKKKKLIGSYKYIG
ASIDMDLATANDGVTYYNKMGELYKTHLDGVKTEIKKVEDDIKKQDEELKKLGNVNSQDSKK
NEFIAKKAELEKYLPFLNSLQKEYESLVSKVNTYTDNLKKVINNCQLEKKEAEITVKKLQDYN
KMDEKLEEYKKSEKKNEVKSSGLLEKLMKSKLIK
**SEQ ID NO 17 (His-tagged p19 from *P. ovale*)**
   **>P6528_His-MSP-1-p19_P.ovale_B140110RC** 
MSHHHHHHHHSMDESKKLLSELLDVDSAQLLSMGSKHKCIDITYPDNAGCYRFSDGREEW
RCLLNFKKVGETCVPNNNPTCAENNGGCDPTADCAESENNKITCTCTGQNESFFEGVFCG
**SEQ ID NO 18 (His-tagged p19 from *P. malariae*)**
   **>P6529_His-MSP-1-p19_P.malariae_B140110RB** 
MSHHHHHHHHSMNESTKIISELLGVDSNALLNISAKHACTETKYPENAGCYRYEDGKEVWR
CLLNYKLVDGGCVEDEEPSCQVNNGGCAPEANCTKGDDNKIVCACNAPYSEPIFEGVFCG
**SEQ ID NO 19 (His-tagged p19 from *P. knowlesi*)**
   **>P6530_His-MSP-1-p19_P.knowlesi_B140110RA**

MSHHHHHHHHSMEEESKKVLSQLLNVQTQMLNMSSAHKCIDTNVPENAACYRYLDGTEE
WRCLLGFKEVGGKCVPASITCEENNGGCAPEAECTMDDKKEVECKCTKEGFEPLFEGVFC
S

The present invention is further illustrated by the following figures and non-limiting examples from which further features, embodiments, aspects and advantages of the present invention may be taken.

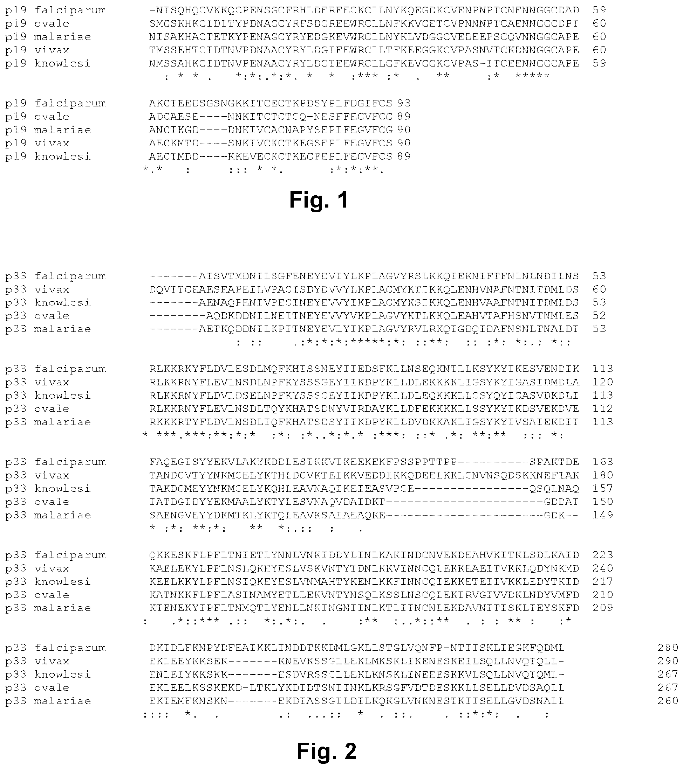

**Fig. 1** shows an alignment of p19 polypeptides from *P. falciparum, P. vivax, P. ovale, P. malariae,* and *P. knowlesi.*
**Fig. 2** shows an alignment of p33 polypeptides from *P. falciparum, P. vivax, P. ovale, P. malariae,* and *P*. *knowlesi.*

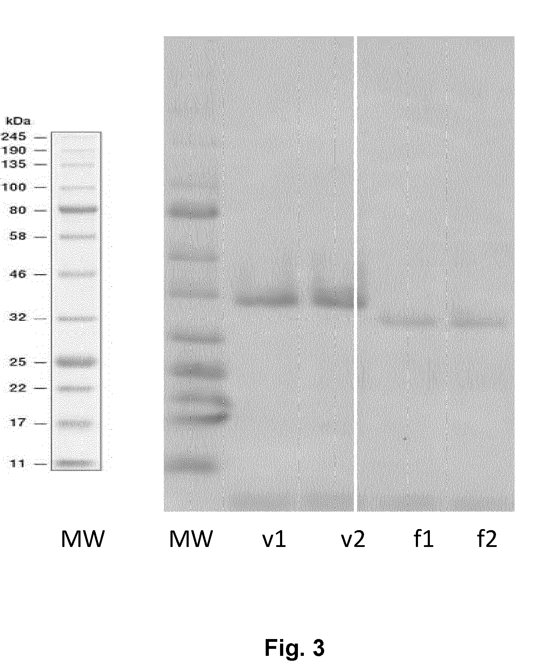

**Fig. 3** shows a molecular weight marker (MW) and purified preparations of p33 polypeptides from *P*. *falciparum* (f1, f2) *and P. vivax* (v1, v2).

### Examples

The following experiments were performed to evaluate the performance of different combinations of *Plasmodium* antigens for the detection of specific *anti-Plasmodium* IgG antibodies in human serum or plasma.

### 1. Samples:

### Panel 1 (sensitivity panel):

The sensitivity panel contained 50 clinical samples of patients with proven *Plasmodium* infection. The causative four *Plasmodium* spp. (*P. falciparum* n=26, *P*. *vivax* n=6, *P. ovale n=12* and *P. malariae* n=6) was determined by microscopy and PCR. Furthermore the clinical samples were analyzed for *anti-Plasmodium* antibodies using the immunofluorescence (IFT) and ELISA.

### Panel 2 (specificity panel):

The specificity panel contains 37 samples which originate from healthy individuals (pregnant woman, children and blood donors from Germany). Previous exposure to *Plasmodium* is highly unlikely because of the origin of the samples.

### 2. Experiments:

### 2.1. Preparation of coated microtiter plates

The following antigens from Merozoite Surface Protein 1 (MSP-1) were used in the form of His-tagged constructs cloned in to vector pET24d (Novagen) and purified by nickel affinity chromatography using standard methodology:
- p19 polypeptide from MSP-1 *P. falciparum* (SEQ ID NO13)
- p33 polypeptide from MSP-1 *P. falciparum* (SEQ ID NO14)
- p19 polypeptide from MSP-1 *P. vivax* (SEQ ID NO15)
- p33 polypeptide from MSP-1 *P. vivax* (SEQ ID NO16)
- p19 polypeptide from MSP-1 *P. ovale* (SEQ ID NO17)
- p19 polypeptide from MSP-1 *P. malariae* (SEQ ID NO18
- p19 polypeptide from MSP-1 *P. knowlesi* (SEQ ID NO19)

For use in microtiter ELISA these antigens were diluted in PBS to final concentrations of approximately 2.0 µg/ml and used to coat ELISA microtiter plates (Nunc, Roskilde, Denmark).

### 2.2. Experimental procedure

Sample were diluted 1:101 in IgG sample buffer, applied to microtiter plates and incubated as described for commercial EUROIMMUN ELISA Test-Kits (e.g. El 2260-9601 G). In brief: 60 min at 37 °C; 3 washing steps using EUROIMMUN washing buffer; addition of 100 µl of peroxidase-labelled anti-human IgG conjugate (goat) per well; incubation for 30 min at 37 °C; 3 washing steps using EUROIMMUN washing buffer; addition of 100 µl of chromogen/substrate solution (TMB/H₂O₂) per well; incubation for 30 min at room temperature; addition of 100 µl stop-solution (0.5 M sulfuric acid); measurement of optical density at 450 nm.

Panel 1 was used for evaluation of sensitivity of the respective antigens for detection of specific anti*-Plasmodium* IgG antibodies. Panel 2 was incubated to determine specificity of the test system.

### 3. Results: Sensitivity and specificity of combinations of p19 and p33 polypeptides of MSP-1 of Plasmodium spp.

Comparison of p19 polypeptides of MSP-1 *P. falciparum, P. vivax, P. ovale, P. malariae* and *P. knowlesi* as well as p33 polypeptides of MSP-1 from *P*. *falciparum* and *P*. *vivax* showed overall sensitivities and specificities of:

| Antibodies detected against | Total sensitivity in % | Total specificity in % |
|---|---|---|
| p19 from *falciparum, vivax, ovale, malariae* (state of the art) | 84.8 | 97.3 |
| p19 from *falciparum, vivax, ovale, malariae* and *knowlesi* | 84.8 | 97.3 |
| p19 from *falciparum, vivax, ovale, malariae* and p33 from *falciparum* | 89.4 | 97.3 |
| p19 from *falciparum, vivax, ovale, malariae* and p33 from *vivax* | 86.7 | 97.3 |
| p19 from *falciparum, vivax, ovale, malariae* and p33 from *falciparum* | 89.4 | 97.3 |
| p19 from *falciparum, vivax, ovale, malariae* and p33 from *vivax* | 86.7 | 97.3 |

The sample set used for evaluation of sensitivity of this test are samples originating from patients with acute infections. If these samples were drawn at early time points of infection there would be the possibility that they lack anti*-Plasmodium* IgG because seroconversion has yet not taken place (four samples positive for PCR and microscopy, but serology is negative.

### Conclusion

Combining the state of the art combination of antigens comprising p19 polypeptides from *P*. *falciparum, P. vivax, P. ovale, P. malariae, optionally* together with p19 from *P*. *knowlesi,* with a p33 polypeptide increases the overall diagnostic sensitivity, while the specificity is not impaired.

## Claims

1. Diagnostically useful carrier comprising all means from the group comprising a means for specifically capturing an antibody to p19 from *P. falciparum* according to SEQ ID NO2,
a means for specifically capturing an antibody to p19 from *P.* vivax according to SEQ ID NO3,
a means for specifically capturing an antibody to p19 from *P. ovale* according to SEQ ID NO4 and
a means for specifically capturing an antibody to p19 from *P. malariae* according to SEQ ID NO5;
and further comprising
a means for specifically capturing an antibody to p33 from *P. falciparum* according to SEQ ID NO8 and/or a means for specifically capturing an antibody to p33 from *P.* vivax according to SEQ ID NO9.

2. The diagnostically useful carrier according to claim 1, further comprising a means for specifically capturing an antibody to p19 from *P. knowlesi* according to SEQ ID NO6.

3. The diagnostically useful carrier according to any of claims 1 to 2, wherein at least two means, preferably all means are spatially separate from each other.

4. The diagnostically useful carrier according to any of claims 1 to 3, wherein the carrier is selected from the group comprising a bead, preferably a magnetic and/or fluorescent bead, a test strip, a microtiter plate, a microarray, a solid polymer preferably derived from cellulose, a blot, preferably from the group comprising western blot, line blot and dot blot, a glass surface, a slide, preferably a glass slide for microscopy, a lateral flow device, a chromatography column, a biochip and a membrane, and is most preferably a microtiter plate.

5. The diagnostically useful carrier according to any of claims 1 to 4, wherein the carrier is a mixture of beads on which the means are coated, wherein preferably each bead is coated with one type of means only.

6. The diagnostically useful carrier according to any of claims 1 to 5, wherein the carrier is an ELISA microtiter plate with wells on which the means are coated, wherein preferably each well comprises one type of means only.

7. A set of carriers comprising all means from the group comprising
a means for specifically capturing an antibody to p19 from *P. falciparum* according to SEQ ID NO2,
a means for specifically capturing an antibody to p19 from *P.* vivax according to SEQ ID NO3,
a means for specifically capturing an antibody to p19 from *P. ovale* according to SEQ ID NO4 and
a means for specifically capturing an antibody to p19 from *P. malariae* according to SEQ ID NO5;
and further comprising
a means for specifically capturing an antibody to p33 from *P. falciparum* according to SEQ ID NO8 and/or a means for specifically capturing an antibody to p33 from *P.* vivax according to SEQ ID NO9,
wherein each means is attached to a separate carrier.

8. A method for the detection of *Plasmodium* antibodies comprising the step detecting in a liquid
all antibodies from the group comprising
an antibody to p19 from *P. falciparum* according to SEQ ID NO2,
an antibody to p19 from *P. vivax* according to SEQ ID NO3,
an antibody to p19 from *P. ovale* according to SEQ ID NO4 and
an antibody to p19 from *P. malariae* according to SEQ ID NO5
and
one or more antibodies, preferably all antibodies, from the group comprising
an antibody to p33 from *P. falciparum* according to SEQ ID NO8 and an antibody to p33 from P. vivax according to SEQ ID NO9.

9. The method according to claim 8, wherein the antibody detection comprises the use of a method selected from the group comprising immunodiffusion techniques, immunoelectrophoretic techniques, light scattering immunoassays, agglutination techniques, labeled immunoassays such as those from the group comprising radiolabeled immunoassays, enzyme immunoassays such as colorimetric assays, chemiluminscence immunoassays and immunofluorescence techniques,
or wherein indirect immunofluorescence is used.

10. The method according to any of claims 8 to 9, wherein at least an antibody to a *Plasmodium* p19 and an antibody to a *Plasmodium* p33 are detected in spatially separate reactions.

11. Use of the diagnostically useful carrier according to any of claims 1 to 6 or the set of carriers according to claim 7 for identifying blood from a healthy blood donor or for detecting a Plasmodium infection in a liquid sample comprising antibodies from a subject.

12. A method for determining the ability, preferably capacity of a carrier according to any of claims 1 to 6 or the set of carriers according to claim 7 to bind to
an antibody to p19 from *P. falciparum* according to SEQ ID NO2,
an antibody to p19 from *P. vivax* according to SEQ ID NO3,
an antibody to p19 from *P. ovale* according to SEQ ID NO4,
an antibody to p19 from *P. malariae* according to SEQ ID NO5,
an antibody to p33 from *P. falciparum* according to SEQ ID NO8 and
an antibody to p33 from *P.* vivax according to SEQ ID NO9,
comprising the step of contacting the diagnostically useful carrier according to any of claims 1 to 6 or the set of carriers according to claim 7 with a solution comprising a known concentration of at least one antibody to a *Plasmodium* p19 and a solution comprising a known concentration of at least one antibody to a *Plasmodium* p33.

13. A kit comprising the diagnostically useful carrier according to any of claims 1 to 6 or the set of carriers according to claim 7 and a means for detecting an antibody to a sequence from the group comprising SEQ ID NO2, SEQ ID NO3, SEQ ID NO4, SEQ ID NO5, SEQ ID NO8 and SEQ ID NO9 when bound to the diagnostically useful carrier or set of carriers, preferably a labeled secondary antibody,
wherein the kit preferably further comprises one or more, preferably all from the group comprising sample dilution buffer, washing buffer, a positive control, preferably a recombinant antibody binding to one or more from the group comprising SEQ ID NO2, SEQ ID NO3, SEQ ID NO4, SEQ ID NO5, SEQ ID NO6, SEQ ID NO8 and SEQ ID NO9, a negative control, one or more standard solutions comprising an antibody binding to one or more from SEQ ID NO2, to SEQ ID NO3, to SEQ ID NO4, to SEQ ID NO5, to SEQ ID NO6, to SEQ ID NO8 and to SEQ ID NO9 for preparing a calibration curve and a suitable water-tight vessel for contacting the diagnostically useful carrier with the sample in the presence of other liquids.

14. A kit comprising the diagnostically useful carrier according to any of claims 1 to 6 or the set of carriers according to claim 7 and a purified polypeptide comprising one sequence from the group comprising SEQ ID NO2, SEQ ID NO3, SEQ ID NO4, SEQ ID NO5, SEQ ID NO8 and SEQ ID NO9, which polypeptide is optionally labeled.

15. The kit according to claim 14, further comprising
a. a positive control and a negative control, and/or
b. one or more standard solutions comprising an antibody binding to one or more from SEQ ID NO2, SEQ ID NO3, SEQ ID NO4, SEQ ID NO5, SEQ ID NO8 and SEQ ID NO9 for preparing a calibration curve, preferably wherein the absolute or relative concentration of the antibody in each standard solution is known, and/or
c. a means or reagent for detecting a captured antibody, and/or
d. a washing solution, and/or
e. a suitable water-tight vessel for contacting the diagnostically useful carrier with the sample in the presence of other liquids.

## Patentansprüche

1. Diagnostisch verwendbarer Träger, umfassend
alle Mittel aus der Gruppe, umfassend
ein Mittel zum spezifischen Einfangen eines Antikörpers gegen p19 aus *P. falciparum* gemäß SEQ ID NO2,
ein Mittel zum spezifischen Einfangen eines Antikörpers gegen p19 aus *P. vivax* gemäß SEQ ID NO3,
ein Mittel zum spezifischen Einfangen eines Antikörpers gegen p19 aus *P. ovale* gemäß SEQ ID NO4 und
ein Mittel zum spezifischen Einfangen eines Antikörpers gegen p19 aus *P. malariae* gemäß SEQ ID NO5;
und ferner umfassend
ein Mittel zum spezifischen Einfangen eines Antikörpers gegen p33 aus *P. falciparum* gemäß SEQ ID NO8 und/oder ein Mittel zum spezifischen Einfangen eines Antikörpers gegen p33 aus *P. vivax* gemäß SEQ ID NO9.

2. Diagnostisch verwendbarer Träger nach Anspruch 1, ferner umfassend ein Mittel zum spezifischen Einfangen eines Antikörpers gegen p19 aus *P. knowlesi* gemäß SEQ ID NO6.

3. Diagnostisch verwendbarer Träger nach einem der Ansprüche 1 bis 2, wobei wenigstens zwei Mittel, vorzugsweise alle Mittel, räumlich voneinander getrennt sind.

4. Diagnostisch verwendbarer Träger nach einem der Ansprüche 1 bis 3, wobei der Träger ausgewählt ist aus der Gruppe, umfassend ein Bead, vorzugsweise ein magnetisches und/oder fluoreszierendes Bead, einen Teststreifen, eine Mikrotiterplatte, einen Microarray, ein festes Polymer, vorzugsweise abgeleitet von Zellulose, einen Blot, vorzugsweise aus der Gruppe, umfassend Western-Blot, Line-Blot und Dot-Blot, eine Glasoberfläche, einen Objektträger, vorzugsweise einen Glasobjektträger für die Mikroskopie, eine Lateral-Flow-Vorrichtung, eine Chromatographiesäule, einen Biochip und eine Membran, und am meisten bevorzugt eine Mikrotiterplatte ist.

5. Diagnostisch verwendbarer Träger nach einem der Ansprüche 1 bis 4, wobei der Träger ein Gemisch aus Beads, auf denen die Mittel beschichtet sind, ist, wobei vorzugsweise ein jegliches Bead mit nur einer Art von Mittel beschichtet ist.

6. Diagnostisch verwendbarer Träger nach einem der Ansprüche 1 bis 5, wobei der Träger eine ELISA-Mikrotiterplatte mit Vertiefungen ist, auf denen die Mittel beschichtet sind, wobei vorzugsweise eine jegliche Vertiefung nur eine Art von Mitteln umfasst.

7. Trägerset, umfassend
alle Mittel aus der Gruppe, umfassend
ein Mittel zum spezifischen Einfangen eines Antikörpers gegen p19 aus *P. falciparum* gemäß SEQ ID NO2,
ein Mittel zum spezifischen Einfangen eines Antikörpers gegen p19 aus *P. vivax* gemäß SEQ ID NO3,
ein Mittel zum spezifischen Einfangen eines Antikörpers gegen p19 aus *P. ovale* gemäß SEQ ID NO4 und
ein Mittel zum spezifischen Einfangen eines Antikörpers gegen p19 aus *P. malariae* gemäß SEQ ID NO5;
und ferner umfassend
ein Mittel zum spezifischen Einfangen eines Antikörpers gegen p33 aus *P. falciparum* gemäß SEQ ID NO8 und/oder ein Mittel zum spezifischen Einfangen eines Antikörpers gegen p33 aus *P. vivax* gemäß SEQ ID NO9,
wobei ein jegliches Mittel an einem separaten Träger angebracht ist.

8. Verfahren zum Nachweisen von Plasmodium-Antikörpern, umfassend den Schritt Nachweisen in einer Flüssigkeit
aller Antikörper aus der Gruppe, umfassend
einen Antikörper gegen p19 aus *P. falciparum* gemäß SEQ ID NO2,
einen Antikörper gegen p19 aus *P. vivax* gemäß SEQ ID NO3,
einen Antikörper gegen p19 aus *P. ovale* gemäß SEQ ID NO4 und
einen Antikörper gegen p19 aus *P. malariae* gemäß SEQ ID NO5,
und
eines oder mehrerer Antikörper/s, vorzugsweise aller Antikörper, aus der Gruppe, umfassend einen Antikörper gegen p33 aus *P. falciparum* gemäß SEQ ID NO8 und einen Antikörper gegen p33 aus *P. vivax* gemäß SEQ ID NO9.

9. Verfahren nach Anspruch 8, wobei der Antikörpernachweis die Verwendung eines Verfahrens, das aus der Gruppe, umfassend Immundiffusionstechniken, immunelektrophoretische Techniken, Lichtstreuungsimmunassays, Agglutinationstechniken, markierte Immunassays, wie solche aus der Gruppe, umfassend radioaktiv markierte Immunassays, Enzym-Immunassays, wie kolorimetrische Assays, Chemilumineszenz-Immunassays und Immunfluoreszenztechniken, ausgewählt ist, umfasst,
oder wobei indirekte Immunfluoreszenz verwendet wird.

10. Verfahren nach einem der Ansprüche 8 bis 9, wobei wenigstens ein Antikörper gegen ein *Plasmodium-*p19 und ein Antikörper gegen ein *Plasmodium-*p33 in räumlich getrennten Reaktionen nachgewiesen werden.

11. Verwendung des diagnostisch verwendbaren Trägers nach einem der Ansprüche 1 bis 6 oder des Trägersets nach Anspruch 7 zum Identifizieren von Blut eines gesunden Blutspenders oder zum Nachweisen einer Plasmodium-Infektion in einer flüssigen Probe, umfassend Antikörper eines Lebewesens.

12. Verfahren zum Bestimmen der Fähigkeit, vorzugsweise Kapazität, eines Trägers nach einem der Ansprüche 1 bis 6 oder des Trägersets nach Anspruch 7 zum Binden an
einen Antikörper gegen p19 aus *P. falciparum* gemäß SEQ ID NO2,
einen Antikörper gegen p19 aus *P. vivax* gemäß SEQ ID NO3,
einen Antikörper gegen p19 aus *P. ovale* gemäß SEQ ID NO4,
einen Antikörper gegen p19 aus *P. malariae* gemäß SEQ ID NO5,
einen Antikörper gegen p33 aus *P. falciparum* gemäß SEQ ID NO8 und
einen Antikörper gegen p33 aus *P. vivax* gemäß SEQ ID NO9,
umfassend den Schritt Inkontaktbringen des diagnostisch verwendbaren Trägers nach einem der Ansprüche 1 bis 6 oder des Trägersets nach Anspruch 7 mit einer Lösung, umfassend eine bekannte Konzentration wenigstens eines Antikörpers gegen ein *Plasmodium-*p19*,* und einer Lösung, umfassend eine bekannte Konzentration wenigstens eines Antikörpers gegen ein *Plasmodium-*p33.

13. Kit, umfassend den diagnostisch verwendbaren Träger nach einem der Ansprüche 1 bis 6 oder das Trägerset nach Anspruch 7 und ein Mittel zum Nachweisen eines Antikörpers gegen eine Sequenz aus der Gruppe, umfassend SEQ ID NO2, SEQ ID NO3, SEQ ID NO4, SEQ ID NO5, SEQ ID NO8 und SEQ ID NO9, wenn sie an den diagnostisch verwendbaren Träger oder das Trägerset gebunden ist, vorzugsweise einen markierten Sekundärantikörper,
wobei das Kit vorzugsweise ferner eines oder mehrere, vorzugsweise alle aus der Gruppe, umfassend einen Probenverdünnungspuffer, einen Waschpuffer, eine Positivkontrolle, vorzugsweise einen rekombinanten Antikörper, der an eines oder mehrere aus der Gruppe, umfassend SEQ ID NO2, SEQ ID NO3, SEQ ID NO4, SEQ ID NO5, SEQ ID NO6, SEQ ID NO8 und SEQ ID NO9, bindet, eine Negativkontrolle, eine oder mehrere Standardlösungen, umfassend einen Antikörper, der an eines oder mehrere von SEQ ID NO2, SEQ ID NO3, SEQ ID NO4, SEQ ID NO5, SEQ ID NO6, SEQ ID NO8 und SEQ ID NO9 bindet, zum Erstellen einer Kalibrierungskurve, und ein geeignetes wasserdichtes Gefäß zum Kontaktieren des diagnostisch verwendbaren Trägers mit der Probe in der Gegenwart anderer Flüssigkeiten, umfasst.

14. Kit, umfassend den diagnostisch verwendbaren Träger nach einem der Ansprüche 1 bis 6 oder das Trägerset nach Anspruch 7 und ein aufgereinigtes Polypeptid, umfassend eine Sequenz aus der Gruppe, umfassend SEQ ID NO2, SEQ ID NO3, SEQ ID NO4, SEQ ID NO5, SEQ ID NO8 und SEQ ID NO9, wobei das Polypeptid gegebenenfalls markiert ist.

15. Kit nach Anspruch 14, ferner umfassend
a. eine Positivkontrolle und eine Negativkontrolle, und/oder
b. eine oder mehrere Standardlösungen, umfassend einen Antikörper, der an eines oder mehrere von SEQ ID NO2, SEQ ID NO3, SEQ ID NO4, SEQ ID NO5, SEQ ID NO8 und SEQ ID NO9 bindet, zum Erstellen einer Kalibrierungskurve, vorzugsweise wobei die absolute oder relative Konzentration des Antikörpers in einer jeglichen Standardlösung bekannt ist, und/oder
c. ein Mittel oder Reagenz zum Nachweisen eines eingefangenen Antikörpers, und/oder
d. eine Waschlösung, und/oder
e. ein geeignetes wasserdichtes Gefäß zum Kontaktieren des diagnostisch verwendbaren Trägers mit der Probe in der Gegenwart anderer Flüssigkeiten.

## Revendications

1. Support utile sur le plan diagnostique comprenant tous les moyens du groupe comprenant
un moyen pour capturer spécifiquement un anticorps contre p19 de P. falciparum selon la SEQ ID NO2,
un moyen pour capturer spécifiquement un anticorps contre p19 de P. vivax selon la SEQ ID NO3,
un moyen pour capturer spécifiquement un anticorps contre p19 de P. ovale selon la SEQ ID NO4 et
un moyen pour capturer spécifiquement un anticorps contre p19 de P. malariae selon la SEQ ID NO5;
et comprenant en outre
un moyen pour capturer spécifiquement un anticorps contre p33 de P. falciparum selon la SEQ ID NO5 et/ou un moyen pour capturer spécifiquement un anticorps contre p33 de P. vivax selon la SEQ ID NO9.

2. Support utile sur le plan diagnostique selon la revendication 1, comprenant en outre un moyen pour capturer spécifiquement un anticorps contre p19 de P. knowlesi selon la SEQ ID NO6.

3. Support utile sur le plan diagnostique selon l'une quelconque des revendications 1 à 2, dans lequel au moins deux moyens, de préférence tous les moyens, sont spatialement distincts les uns des autres.

4. Support utile sur le plan diagnostique selon l'une quelconque des revendications 1 à 3, dans lequel le support est choisi dans le groupe comprenant une bille, de préférence une bille magnétique et/ou fluorescente, une bande de test, une plaque de microtitrage, un microréseau, un polymère solide de préférence dérivé de cellulose, un transfert, de préférence du groupe comprenant Western blot, line blot et dot blot, une surface de verre, une lame, de préférence une lame de verre pour microscopie, un dispositif à flux latéral, une colonne de chromatographie, une biopuce et une membrane, et est de préférence une plaque de microtitrage.

5. Support utile sur le plan diagnostique selon l'une quelconque des revendications 1 à 4, dans lequel le support est un mélange de billes sur lesquelles les moyens sont revêtus, dans lequel de préférence chaque bille est revêtue par seulement un type de moyens.

6. Support utile sur le plan diagnostique selon l'une quelconque des revendications 1 à 5, dans lequel le support est une plaque de microtitrage ELISA avec des puits sur lesquels les moyens sont revêtus, dans lequel de préférence chaque puits comprend seulement un type de moyens.

7. Ensemble de supports comprenant tous les moyens du groupe comprenant un moyen pour capturer spécifiquement un anticorps contre p19 de P. falciparum selon la SEQ ID NO2,
un moyen pour capturer spécifiquement un anticorps contre p19 de P. vivax selon la SEQ ID NO3,
un moyen pour capturer spécifiquement un anticorps contre p19 de P. ovale selon la SEQ ID NO4 et
un moyen pour capturer spécifiquement un anticorps contre p19 de P. malariae selon la SEQ ID NO5;
et comprenant en outre
un moyen pour capturer spécifiquement un anticorps contre p33 de P. falciparum selon la SEQ ID NO8 et/ou un moyen pour capturer spécifiquement un anticorps contre p33 de P. vivax selon la SEQ ID NO9,
dans lequel chaque moyen est fixé sur un support distinct.

8. Procédé de détection d'anticorps de Plasmodium comprenant l'étape de détection dans un liquide de
tous les anticorps du groupe comprenant
un anticorps contre p19 de P. falciparum selon la SEQ ID NO2,
un anticorps contre p19 de P. vivax selon la SEQ ID NO3,
un anticorps contre p19 de P. ovale selon la SEQ ID NO4 et
un anticorps contre p19 de P. malariae selon la SEQ ID NO5
et
un ou plusieurs anticorps, de préférence tous les anticorps, du groupe comprenant
un anticorps contre p33 de P. falciparum selon la SEQ ID NO8 et un anticorps contre p33 de P. vivax selon la SEQ ID NO9.

9. Procédé selon la revendication 8, dans lequel la détection d'anticorps comprend l'utilisation d'un procédé choisi dans le groupe comprenant les techniques d'immunodiffusion, les techniques immunoélectrophorétiques, les dosages immunologiques par diffusion de la lumière, les techniques d'agglutination, les dosages immunologiques marqués tels que ceux du groupe comprenant les dosages immunologiques radiomarqués, les dosages immunologiques enzymatiques tels que les dosages colorimétriques, les dosages immunologiques par chimioluminescence et les techniques d'immunofluorescence,
ou dans lequel une immunofluorescence indirecte est utilisée.

10. Procédé selon l'une quelconque des revendications 8 à 9, dans lequel au moins un anticorps contre un Plasmodium p19 et un anticorps contre un Plasmodium p33 sont détectés dans des réactions spatialement distinctes.

11. Utilisation du support utile sur le plan diagnostique selon l'une quelconque des revendications 1 à 6 ou de l'ensemble de supports selon la revendication 7 pour identifier du sang provenant d'un donneur de sang sain ou pour détecter une infection par Plasmodium dans un échantillon liquide comprenant des anticorps provenant d'un sujet.

12. Procédé pour déterminer l'aptitude, de préférence la capacité, d'un support selon l'une quelconque des revendications 1 à 6 ou de l'ensemble de supports selon la revendication 7 à se lier à
un anticorps contre p19 de P. falciparum selon la SEQ ID NO2,
un anticorps contre p19 de P. vivax selon la SEQ ID NO3,
un anticorps contre p19 de P. ovale selon la SEQ ID NO4,
un anticorps contre p19 de P. malariae selon la SEQ ID NO5,
un anticorps contre p33 de P. falciparum selon la SEQ ID NO8 et
un anticorps contre p33 de P. vivax selon la SEQ ID NO9,
comprenant l'étape de mise en contact du support utile sur le plan diagnostique selon l'une quelconque des revendications 1 à 6 ou de l'ensemble de supports selon la revendication 7 avec une solution comprenant une concentration connue d'au moins un anticorps contre un Plasmodium p19 et une solution comprenant une concentration connue d'au moins un anticorps contre un Plasmodium p33.

13. Kit comprenant le support utile sur le plan diagnostique selon l'une quelconque des revendications 1 à 6 ou l'ensemble de supports selon la revendication 7 et un moyen pour détecter un anticorps contre une séquence du groupe comprenant SEQ ID NO2, SEQ ID NO3, SEQ ID NO4, SEQ ID NO5, SEQ ID NO8 et SEQ ID NO9 lorsqu'il est lié au support ou l'ensemble de supports utile sur le plan diagnostique, de préférence un anticorps secondaire marqué,
dans lequel le kit comprend de préférence en outre un ou plusieurs, de préférence tous dans le groupe comprenant un tampon de dilution d'échantillon, un tampon de lavage, un témoin positif, de préférence un anticorps recombinant se liant à un ou plusieurs dans le groupe comprenant SEQ ID NO2, SEQ ID NO3, SEQ ID NO4, SEQ ID NO5, SEQ ID NO6, SEQ ID NO8 et SEQ ID NO9, un témoin négatif, une ou plusieurs solutions standard comprenant un anticorps se liant à un ou plusieurs parmi SEQ ID NO2, SEQ ID NO3, SEQ ID NO4, SEQ ID NO5, SEQ ID NO6, SEQ ID NO8 et SEQ ID NO9 pour préparer une courbe d'étalonnage et un récipient étanche à l'eau approprié pour mettre en contact le support utile sur le plan diagnostique avec l'échantillon en présence d'autres liquides.

14. Kit comprenant le support utile sur le plan diagnostique selon l'une quelconque des revendications 1 à 6 ou l'ensemble de supports selon la revendication 7 et un polypeptide purifié comprenant une séquence du groupe comprenant SEQ ID NO2, SEQ ID NO3, SEQ ID NO4, SEQ ID NO5, SEQ ID NO8 et SEQ ID NO9, lequel polypeptide est facultativement marqué.

15. Kit selon la revendication 14, comprenant en outre
a. un témoin positif et un témoin négatif, et/ou
b. une ou plusieurs solutions standard comprenant un anticorps se liant à un ou plusieurs parmi SEQ ID NO2, SEQ ID NO3, SEQ ID NO4, SEQ ID NO5, SEQ ID NO8 et SEQ ID NO9 pour préparer une courbe d'étalonnage, de préférence dans lequel la concentration absolue ou relative de l'anticorps dans chaque solution standard est connue, et/ou
c. un moyen ou un réactif pour détecter un anticorps capturé, et/ou
d. une solution de lavage, et/ou
e. un récipient étanche à l'eau approprié pour mettre en contact le support utile sur le plan diagnostique avec l'échantillon en présence d'autres liquides.
